# EUROPEAN PATENT APPLICATION

(11) **EP 3 932 251 A1**
(43) Date of publication of application: **05.01.2022**
(21) Application number: 19916739.6
(22) Date of filing: 12.11.2019
(51) Int. Cl.: A45D 44/22, A61F 13/00, A61F 13/02, G06T 1/00

(54) **IMAGE PROCESSING DEVICE, IMAGE PROCESSING METHOD AND SHEET**

(30) Priority: 28.02.2019 JP 2019035697
(71) Applicant: Panasonic Intellectual Property Management Co., Ltd., Osaka-shi, Osaka 540-6207 (JP)
(72) Inventor: SHINODA, Masayo, Osaka-shi, Osaka (JP); UEDA, Mari, Osaka-shi, Osaka (JP); TAOKA, Hiroki, Osaka-shi, Osaka (JP); TAKESHITA, Sachiko, Osaka-shi, Osaka (JP)
(74) Representative: Grünecker Patent- und Rechtsanwälte PartG mbB
(86) International application number: PCT/JP2019/044260
(87) International publication number: WO 2020/174766

(57) **Abstract**

An image processing apparatus is provided which produces image data to make a discolored portion having a lightness higher than that of a periphery thereof inconspicuous. The image processing apparatus includes: an image analysis section which identifies a discolored region corresponding to the discolored portion from a photographed image of the discolored portion; and an image data production section which produces, based on information of a color of a normal region of the image corresponding to a normal portion and a color of the discolored region, the image data to correct a color of the discolored portion to a color of the normal portion. The image data includes a first layer located at a skin side, a second layer located on the first layer, and a third layer located on the second layer. The image data production section sets an L* value of a region of the first layer corresponding to the discolored region to a value lower than an L* value of the normal region by higher than or equal to 5 and lower than or equal to 20.

## Description

### Technical Field

The present disclosure relates to an image processing apparatus, an image processing method, and a sheet.

### Background Art

Heretofore, there has been proposed a technique in which a sheet is produced by applying at least one ink containing various types of colorants on a thin film and is then adhered to a human body so as to make a discolored portion, such as a spot, a blotch, or a scar, generated on her or his skin inconspicuous (for example, see Patent Literature 1). According to the technique disclosed in Patent Literature 1, the skin is photographed, and the discolored portion is identified. In addition, according to the technique described above, it is believed that since a color similar to that of a periphery of the discolored portion is printed on a thin film to produce the sheet, and this sheet is adhered to a skin, the discolored portion can be made inconspicuous.

### Citation List

### Patent Literature

Patent Literature 1: Japanese Unexamined Patent Application Publication No. 2015-43836

### Summary of Invention

However, according to the technique disclosed in Patent Literature 1, since the color of the discolored portion is hidden and is matched with that of the periphery thereof, the color of the periphery of the discolored portion is printed dark. However, in the case in which the sheet is formed as described above, a thick coating feeling is liable to be generated when the sheet is adhered, and on the contrary, the discolored portion unfavorably tends to be conspicuous.

In addition, among various types of skin discolored portions, for example, there may be mentioned a discolored portion, such as a spot or a blotch, having a lightness (low L* value) lower than that of a periphery thereof and a discolored portion, such as a vitiligo, having a lightness (high L* value) higher than that of a periphery thereof. The discolored portion having a lightness higher than that of the periphery thereof may have a clear outline in many cases. Hence, by the technique disclosed in Patent Literature 1, the discolored portion having a lightness higher than that of the periphery thereof cannot be appropriately made inconspicuous.

The present disclosure provides an image processing apparatus and an image processing method each of which produces image data to make a discolored portion having a lightness higher than that of a periphery thereof inconspicuous. In addition, the present disclosure also provides a sheet which makes a discolored portion having a lightness higher than that of a periphery thereof inconspicuous.

An image processing apparatus according to an embodiment of the present disclosure is an image processing apparatus which produces image data to correct a color of a discolored portion having a lightness higher than that of a normal portion of a skin to a color of the normal portion, the image processing apparatus comprising: an image analysis section which identifies a discolored region corresponding to the discolored portion from a photographed image of the discolored portion; and an image data production section which produces, based on information of a color of a normal region of the image corresponding to the normal portion and a color of the discolored region, the image data to correct the color of the discolored portion to the color of the normal portion. In the image processing apparatus described above, the image data includes a first layer located at a skin side, a second layer located on the first layer, and a third layer located on the second layer, and the image data production section sets an L* value of a region of the first layer corresponding to the discolored region to a value lower than an L* value of the normal region by higher than or equal to 5 and lower than or equal to 20.

The image processing apparatus, the image processing method, and the sheet according to the present disclosure are each able to make a discolored portion having a lightness higher than that of a periphery thereof inconspicuous without generating a thick coating feeling.

### Brief Description of Drawings

[Fig. 1] Fig. 1 is a block diagram showing the structure of an image processing apparatus.
[Fig. 2] Fig. 2 is a schematic view showing one example of an image processing apparatus which can form a sheet.
[Fig. 3] Fig. 3 is a view showing a hardware structure of a computer which realizes a function of the image processing apparatus by a program.
[Fig. 4] Fig. 4 is a schematic cross-sectional view showing the structure of the sheet.
[Fig. 5] Fig. 5 is a flowchart showing an operation of the image processing apparatus.
[Fig. 6] Fig. 6 is a flowchart showing one example of processing of an image analysis section in Step S102.
[Fig. 7] Figs. 7A to D are each a photo showing a result of Experiment 1.
[Fig. 8] Figs. 8A to C are each a photo showing a result of Experiment 2. Description of Embodiments

An image processing apparatus according to the present disclosure produces image data to correct a color of a discolored portion having a lightness higher than that of a normal portion of a skin to a color of the normal portion. The image processing apparatus according to the present disclosure may further have, based on the image data described above, a function to form on a skin or a thin film, an image which corrects the color of the discolored portion to the color of the normal portion. The image processing apparatus as described above is also called a makeup support system.

In the present disclosure, a correction object is a discolored portion having a lightness higher than that of a peripheral normal portion (non-discolored portion). As an example of the discolored portion of the skin as described above, for example, there may be mentioned a vitiligo, a skin grafted portion to which a skin having a lightness higher than that of a periphery thereof is grafted, a non-sunburnt region surrounded by a sunburnt region, a portion (such as a portion having a color irregularity or a portion formed by an operator's mistake) unintentionally having a lightness higher than that of a periphery thereof by body painting or the like, a non-makeup portion surrounded by a makeup portion, or a makeup- portion surrounded by a non-makeup portion.

In addition, in the following description, "L* value", "a* value", and "b* value" represent the L* value, the a* value, and the b* value of the L*a*b* color system (CIE1976), respectively.

### (STRUCTURE OF IMAGE PROCESSING APPARATUS)

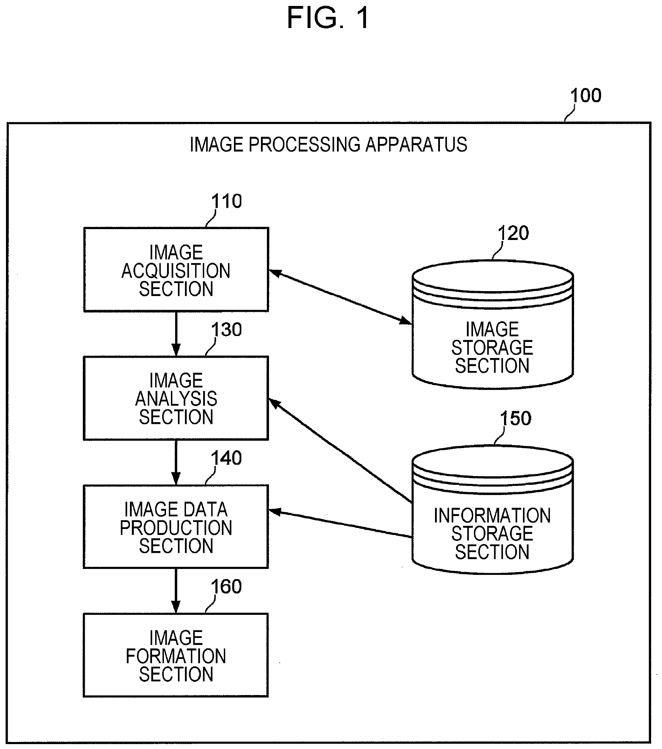

Fig. 1 is a block diagram showing the structure of an image processing apparatus (makeup support system) 100 according to an embodiment of the present disclosure. As shown in Fig. 1, the image processing apparatus (makeup support system) 100 includes an image acquisition section 110, an image storage section 120, an image analysis section 130, an image data production section 140, an information storage section 150, and an image formation section 160.

The image acquisition section 110 acquires an image of a skin including a discolored portion. For example, the image acquisition section 110 photographs a user's face by a camera. Alternatively, the image acquisition section 110 may acquire an image photographed by an external apparatus by input therefrom or an image stored in the image storage section 120 by input therefrom. The image acquisition section 110 transmits the acquired image to the image analysis section 130 and, if needed, also stores the acquired image in the image storage section 120.

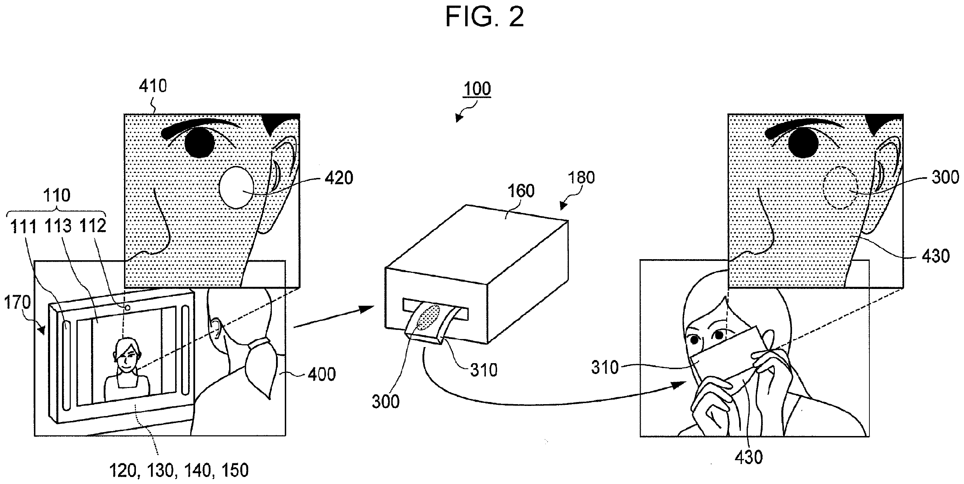

For example, the image acquisition section 110 includes lighting which radiates visible light rays, a camera, and a display portion, such as a touch-panel equipped liquid crystal display, which also functions as an input portion (see Fig. 2). The lighting and the camera are preferably disposed in the vicinity of the display portion. In this case, while visible light rays are radiated by the lighting on the user's face, the image acquisition section 110 photographs the user's face located at a front side of the display portion by the camera. In addition, after the image thus photographed is reversed from left to right, the image acquisition section 110 displays the image on the display portion. Accordingly, while feeling as if looking in a mirror, the user is able to photograph his or her face.

In the image storage section 120, the image acquired by the image acquisition section 110 is stored.

The image analysis section 130 identifies a discolored region corresponding to the discolored portion from the image acquired by the image acquisition section 110. In addition, the image analysis section 130 also identifies a normal region corresponding to the normal portion in the periphery of the discolored portion from the same image. In addition, the image analysis section 130 identifies a shape and a color of the discolored region and also identifies a color of the normal region. The image analysis section 130 transmits the information thus identified to the image data production section 140. When the image is analyzed, if needed, the image analysis section 130 may use various types of information (such as a first threshold value and a second threshold value, each of which will be described later) stored in the information storage section 150.

A method in which the image analysis section 130 identifies the discolored region is not particularly limited. For example, the image analysis section 130 may identify the discolored region by allowing the user to designate a range of the discolored region in the image using the display portion, such as the touch-panel equipped liquid crystal display described above, which also functions as the input portion (see Fig. 2). Alternatively, photographing and image acquisition may be performed by a probe connected to the display portion.

In addition, the image analysis section 130 may identify in the image, a region in which a lightness (such as the L* value) is the first threshold value or more as the discolored region. The first threshold value is not particularly limited and may be appropriately determined by a skin color in a normal region (non-discolored region) of the user. For example, the first threshold value is in a range of 60 to 100 as the L* value. In addition, in the case described above, when a region in which the lightness is the first threshold value or more is present, although the image analysis section 130 may immediately identify this region as the discolored region, in order to improve identification accuracy of the discolored region, only when other conditions are also satisfied, this region may be identified as the discolored region.

For example, only when an area of 30% or more of an outer edge of the region in which the lightness (L* value) is the first threshold value or more is surrounded by a region (normal region) in which the lightness (L* value) is less than the first threshold value, the image analysis section 130 may identify this region as the discolored region. Accordingly, the identification accuracy of the discolored region with respect to a user (a fair user) having an entire skin of high lightness can be improved. In addition, only when a b* value in the region in which the lightness (L* value) is the first threshold value or more is lower than a b* value in the region in which the lightness (L* value) is less than the first threshold value by 5 or more, the image analysis section 130 may identify this region as the discolored region. Accordingly, the identification accuracy of the discolored region can also be improved.

In addition, in the region in which the lightness (L* value) is the first threshold value or more, only when a plurality of regions in each of which the L* value is less than the first threshold value is not present at intervals of a predetermined second threshold value or less, that is, only when spots or the like are not present in this region, the image analysis section 130 may identify this region as the discolored region. Accordingly, the identification accuracy of the discolored region with respect to a user having an entire skin of high lightness can be improved. In addition, in the region in which the lightness (L* value) is the first threshold value or more, when a plurality of regions in each of which the L* value is less than the first threshold value is present at intervals of the predetermined second threshold value or less, the plurality of regions in each of which the L* value is less than the first threshold value present at the intervals of the second threshold value or less is identified as spots or the like, and the image analysis section 130 may identify the shape and the like of the discolored region other than those regions described above.

In addition, the image analysis section 130 may identify in the image, a region in which a saturation (such as the S value in the HSV color space) is a predetermined first threshold value or less as the discolored region. As is the case of the lightness, the first threshold value is not particularly limited and may be appropriately determined in accordance with the color or the like of the skin of the normal region (non-discolored region) of the user. In addition, in the case described above, when a region in which the saturation is the first threshold value or less is present, although the image analysis section 130 may immediately identify this region as the discolored region, in order to improve the identification accuracy of the discolored region, only when other conditions are also satisfied, the region described above may be identified as the discolored region.

For example, only when an area of 30% or more of an outer edge of the region in which the saturation (S value) is the first threshold value or less is surrounded by a region (normal region) in which the saturation (S value) is more than the first threshold value, the image analysis section 130 may identify this region as the discolored region. Accordingly, the identification accuracy of the discolored region with respect to a user (a fair user) having an entire skin of low saturation can be improved.

In addition, in the region in which the saturation (S value) is the first threshold value or less, only when a plurality of regions in each of which the S value is more than the first threshold value is not present at intervals of a predetermined second threshold value or less, that is, only when spots or the like are not present in the region described above, the image analysis section 130 may identify this region as the discolored region. Accordingly, the identification accuracy of the discolored region with respect to a user having an entire skin of low saturation can be improved. In addition, in the region in which the saturation (S value) is the first threshold value or less, when a plurality of regions in each of which the S value is more than the first threshold value is present at intervals of the predetermined second threshold value or less, the image analysis section 130 identifies the plurality of regions in each of which the S value is higher than the first threshold value present at the intervals of the second threshold value or less as spots or the like and may also identify the shape or the like of the discolored region other than those regions described above.

In addition, the image analysis section 130 may identify a region in which a spectral reflection spectrum in a visible light wavelength range (400 nm to 700 nm) is uniformly higher than a spectral reflection spectrum of the normal region as the discolored region.

Based on the information (such as the information of the color of the normal region of the image and the information of the shape and the color of the discolored region) obtained from the image analysis section 130 and various types of information stored in the information storage section 150, the image data production section 140 produces image data to correct the color of the discolored portion to the color of the normal portion. As described later, since the image formed based on this image data is disposed on the discolored portion of the user's skin, the discolored portion can be made inconspicuous without generating a thick coating feeling. The image data production section 140 transmits the produced image data to the image formation section 160.

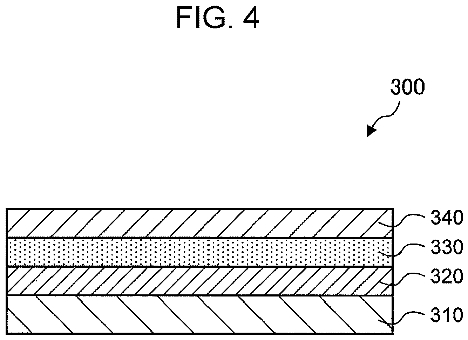

As described later, the image formation section 160 forms an image including at least three layers, that is, a first layer located closest to a skin side, a second layer located on the first layer, and a third layer located on the second layer (see a first layer 320, a second layer 330, and a third layer 340 shown in Fig. 4). In accordance with the image described above, the image data production section 140 also produces image data including at least three layers, that is, a first layer located closest to a skin side, a second layer located on the first layer, and a third layer located on the second layer. The first layer is primarily responsible to have a function so that the lightness of the discolored portion is made similar to that of the normal portion. Since the first layer has a dark hue as compared to that of the peripheral normal portion, the second layer is primarily responsible to have a function so that the hue of the image is made similar to that of the normal portion. The third layer is primarily responsible to have a function so that the appearance of the image is made approximately the same as that of the peripheral normal portion. When the image formed based on the produced image data is disposed on the discolored portion of the user's skin, the image data production section 140 preferably produces image data so that the color difference, ΔE*ab, between the discolored portion and the peripheral normal portion (non-discolored portion) is 3.2 or less, preferably 2.5 or less, and more preferably 1.5 or less.

As described above, the first layer is primarily responsible to have a function so that the lightness of the discolored portion having a lightness higher than that of the peripheral normal portion is made similar to that of the peripheral normal portion. Hence, the image data production section 140 sets the L* value of the region of the first layer corresponding to the discolored region to a value lower than the L* value of the normal region by higher than or equal to 5 and lower than or equal to 20 and preferably by higher than or equal to 10 and lower than or equal to 20. Accordingly, the discolored portion having a high lightness can be effectively shielded. Although the chromaticity (such as the a* value and the b* value) of the region of the first layer corresponding to the discolored region is not particularly limited, the chromaticity described above is preferably substantially the same as the chromaticity of the peripheral normal region. The substantially the same chromaticity between the two regions indicates, for example, that the difference (absolute value) in a* value between the two regions and the difference (absolute value) in b* value between the two regions are each 3.2 or less and preferably 1.5 or less.

As described above, the second layer is primarily responsible to have a function so that the hue of the image is made similar to that of the normal portion. Hence, when the second layer is laminated on the first layer having a dark hue, the image data production section 140 preferably sets the chromaticity (such as the a* value and the b* value) and/or the lightness (such as the L* value) of the region of the second layer corresponding to the discolored region so that the hue of a laminated image is made similar to the hue of the normal portion. For example, the image data production section 140 may set the chromaticity (such as the a* value and the b* value) of the region of the second layer corresponding to the discolored region to a chromaticity substantially having a complementary color relationship with that of the region of the first layer corresponding to the discolored region. In this case, chromaticities having a complementary color relationship with each other indicate two colors facing each other along a straight line passing through the white point in the chromaticity diagram with the white point interposed therebetween. In other words, two different colors which form an achromatic color (such as white, gray, or black) by mixing together at an appropriate ratio are called to have a complementary color relationship. In addition, when color A which is a part of a spectrum is shielded, color B formed by collecting the remaining light has a complementary color relationship with the color A. In addition, in the present disclosure, it is to be understood that the chromaticity having substantially a complementary color relationship with the region of the first layer corresponding to the discolored region includes not only a perfect complementary color of the region of the first layer corresponding to the discolored region but also a chromaticity of a color approximately similar to the above complementary color. In addition, the image data production section 140 may set the lightness (such as the L* value) of the region of the second layer corresponding to the discolored region to a lightness between the lightness of the region of the first layer corresponding to the discolored region and the lightness of the region of the third layer corresponding to the discolored region. For example, the image data production section 140 may set the lightness (such as the L* value) of the region of the second layer corresponding to the discolored region in a range of (an intermediate value between the L* value of the region of the first layer corresponding to the discolored region and the L* value of the region of the third layer corresponding to the discolored region)±(an absolute value of the difference between the L* value of the region of the first layer or the third layer corresponding to the discolored region and the intermediate value described above). In addition, the image data production section 140 may also set the lightness (such as the L* value) of the region of the second layer corresponding to the discolored region in a range in which (the intermediate value-1.5) or (the intermediate value+1.5) is regarded as a lower limit, and a value obtained by adding (the absolute value of the difference between the L* value of the region of the first layer or the third layer corresponding to the discolored region and the intermediate value described above) to the above lower limit is regarded as an upper limit. The range described above is particularly effective when the lightness of the normal portion is low (such as an L* value of less than 65). In addition, the image data production section 140 may set the lightness (such as the L* value) of the region of the second layer corresponding to the discolored region to a lightness higher than the lightness of the region of the first layer corresponding to the discolored region and the lightness of the region of the third layer corresponding to the discolored region. Accordingly, the discolored portion darkened by the first layer is made brighter by the second layer and then can be likely to be corrected to the color of the normal portion by the third layer. The case described above is particularly effective when the lightness of the normal portion is high (such as an L* value of 65 or more).

As described above, the third layer is primarily responsible to have a function so that the appearance of the image is made approximately the same as that of the peripheral normal portion. Hence, the image data production section 140 preferably sets so that the lightness (such as the L* value) and the chromaticity (such as the a* value and the b* value) of the region of the third layer corresponding to the discolored region are substantially the same as the lightness and the chromaticity of the normal region. Accordingly, when the image is placed on the discolored portion, the discolored portion can be made inconspicuous. The case in which the lightness and the chromaticity in one region are substantially the same as those in the other region indicates, for example, that the difference (absolute value) in the L* value between the two regions, the difference (absolute value) in the a* value between the two regions, and the difference (absolute value) in the b* value between the two regions are each 3.2 or less and preferably 1.5 or less. In addition, the case in which the lightness and the chromaticity in one region are substantially the same as those in the other region indicates, for example, that the color difference, ΔE*ab, between the two regions is 3.2 or less, preferably 2.5 or less, and more preferably 1.5 or less.

In addition, in order to make the boundary between the region corresponding to the discolored region and the region corresponding to the peripheral normal region vague, in at least one of the first layer, the second layer, and the third layer, which form the image, the image data production section 140 may set a gradation region between the region corresponding to the discolored region and the region corresponding to the peripheral normal region so that a color is continuously changed from a color of the region corresponding to the discolored region to a color of the region corresponding to the normal region. Unlike a discolored portion, such as a spot, having a low lightness, a discolored portion, such as a vitiligo, having a high lightness has a clear outline in many cases. However, when the gradation region is formed as described above, the outline of the discolored portion having a high lightness can be made more inconspicuous.

For example, the image data production section 140 may also set in the first layer, a gradation region between the region corresponding to the discolored region and the region corresponding to the normal region so that a color is continuously changed from a color in the region corresponding to the discolored region to a color of the region corresponding to the normal region.

In addition, in the second layer or the third layer besides in the first layer, the image data production section 140 may also set a gradation region between the region corresponding to the discolored region and the region corresponding to the normal region so that a color is continuously changed from a color of the region corresponding to the discolored region to a color of the region corresponding to the normal region. In this case, a width of the gradation region in the second layer or the third layer is preferably larger than a width of the gradation region in the first layer. In addition, an inner edge of the gradation region in the second layer or the third layer is preferably located outside than an inner edge of the gradation region in the first layer. Since the gradation regions are set as described above, the outline of the discolored portion can be made more inconspicuous.

In the information storage section 150, various types of information necessary for the image analysis by the image analysis section 130 and various types of information necessary for the production of the image data by the image data production section 140 are stored in advance.

The image formation section 160 forms an image on a skin or a thin film based on the image data produced by the image data production section 140. Since this image is placed on the discolored portion, the discolored portion can be made inconspicuous without generating a thick coating feeling (see Fig. 2).

A method in which the image formation section 160 forms an image is not particularly limited and may be appropriately selected in accordance with an object from which the image is formed. For example, when the image formation section 160 forms an image directly on a region including a skin discolored portion, the image formation section 160 may form the image by either a spray coating or an ink jet printing. When the image is formed directly on the skin as described above, the image can be easily formed for a short time. In addition, when the image formation section 160 forms the image on a thin film which can be adhered to the skin, the image formation section 160 may print the image (the first layer, the second layer, and the third layer) on the thin film. Since the image is formed on the thin film which can be adhered to the skin as described above, a sheet to be adhered to the discolored portion can be produced (see Fig. 2). When the sheet is adhered to the user's skin as described above, compared to the case in which the image is directly formed on the skin, irritation to the skin can be reduced.

When the image formation section 160 forms the image in the region including the skin discolored portion by a spray method, the image formation section 160 forms the first layer, the second layer, and the third layer on the user's skin by sequentially applying inks containing colorants by a spray method. In more particular, the image formation section 160 forms the first layer on the user's skin by spraying a first ink containing a predetermined amount of a predetermined colorant, followed by drying. Subsequently, the image formation section 160 forms the second layer on the first layer by spraying a second ink containing a predetermined amount of a predetermined colorant, followed by drying. Next, the image formation section 160 forms the third layer on the second layer by spraying a third ink containing a predetermined amount of a predetermined colorant, followed by drying. The image formed as described above can be removed by a cleansing agent in a manner similar to that of a general makeup. The ink containing a colorant will be described later.

In the case in which the image formation section 160 performs a spray coating on the user's skin, the structure of the image formation section 160 is not particularly limited. For example, the image formation section 160 includes a fixing stage to fix a user's face, ink storage portions to store at least two types of inks, an ink mixing portion to mix the inks in the ink storage portions, an ink storage chamber to receive an ink mixed in the ink mixing portion, a nozzle to spray the ink in the ink storage chamber, and a control portion to control all the portions described above. The control portion allows, based on the image data produced by the image data production section 140, the ink mixing portion to mix inks having hues necessary to form the image. The nozzle sprays the ink in the ink storage chamber using compressed air. In this case, the control portion adjusts the position of the nozzle, the intensity of air pressure, and the like, so that a desired image is formed. In addition, the image formation section 160 may have a plurality of nozzles, such as a nozzle of an ink containing a colorant for color expression, a nozzle of an ink containing lame and/or pearl for texture expression, and a nozzle of an ink containing an oil or a moisturizer.

When the image formation section 160 forms the image in the region including the skin discolored portion by an ink jet method, the image formation section 160 forms the first layer, the second layer, and the third layer by sequentially printing inks containing colorants on the user's skin by an ink jet method. In more particular, the image formation section 160 forms the first layer on the user's skin by ejecting liquid droplets of a first ink containing a predetermined amount of a predetermined colorant, followed by drying. Subsequently, the image formation section 160 forms the second layer on the first layer by ejecting liquid droplets of a second ink containing a predetermined amount of a predetermined colorant, followed by drying. Next, the image formation section 160 forms the third layer on the second layer by ejecting liquid droplets of a third ink containing a predetermined amount of a predetermined colorant, followed by drying. When the layers are formed, a desired color may be reproduced by using a plurality of inks having different colors in combination. The image formed as described above can be removed by a cleansing agent in a manner similar to that of a general makeup. The ink containing a colorant will be described later.

In the case in which the image formation section 160 performs an ink jet printing on the user's skin, the structure of the image formation section 160 is not particularly limited. For example, the image formation section 160 includes a fixing stage to fix a user's face, ink storage portions to store at least two types of inks, an ink jet head to eject liquid droplets of the inks in the ink storage portions, and a control portion to control all the portions described above. The ink jet head ejects the liquid droplets of the inks in the ink storage portions by a piezoelectric method or a thermal method. In this case, the control portion adjusts the sizes of the liquid droplets of the various types of inks, the positions thereof, the combination therebetween, and the like, so that a desired image is formed. In addition, the image formation section 160 may have a plurality of ink jet heads, such as an ink jet head of an ink containing a colorant for color expression, an ink jet head of an ink containing lame and/or pearl for texture expression, and an ink jet head of an ink containing an oil or a moisturizer.

When the image formation section 160 produces a sheet by forming an image on a thin film which can be adhered to a skin, the image formation section 160 forms the sheet such that the first layer, the second layer, and the third layer are formed by sequentially applying inks containing colorants on the thin film. In more particular, the image formation section 160 forms the first layer on the thin film by applying a first ink containing a predetermined amount of a predetermined colorant, followed by drying. Subsequently, the image formation section 160 forms the second layer on the first layer by applying a second ink containing a predetermined amount of a predetermined colorant, followed by drying. Next, the image formation section 160 forms the third layer on the second layer by applying an third ink containing a predetermined amount of a predetermined colorant, followed by drying. When the layers are formed, a desired color may be reproduced by using inks having different colors in combination.

As shown in Fig. 4, a sheet 300 includes a thin film 310 having one surface to be adhered to a skin, a first layer 320 disposed on the other surface of the thin film 310, a second layer 330 disposed on the first layer 320, and a third layer 340 disposed on the second layer 330. As described above, the L* value of the region of the first layer 320 corresponding to the discolored portion is lower than the L* value of the normal portion by higher than or equal to 10 and lower than or equal to 20, and the lightness and the chromaticity of the region of the third layer 340 corresponding to the discolored portion are substantially the same as the lightness and the chromaticity of the normal portion. In addition, the chromaticity of the region of the second layer 330 corresponding to the discolored region is preferably a chromaticity substantially having a complementary color relationship with that of the region of the first layer 320 corresponding to the discolored region, and the lightness of the region of the second layer 330 corresponding to the discolored region is preferably substantially an intermediate lightness between the lightness of the region of the first layer 320 corresponding to the discolored region and the lightness of the region of the third layer 340 corresponding to the discolored region.

The thin film 310 is preferably a sheet-shaped member which generates no uncomfortable feeling even if being adhered to a human's skin and which has a biocompatible property. In addition, the thin film 310 may be colored as long as the aim and the effect of the present disclosure are not deteriorated or may be colorless transparent or translucent. The thickness of the thin film 310 is preferably 10 nm to 10 µm. When the thin film 310 has a hydrophobic property, the thickness of the thin film 310 is more preferably 10 nm to 1,000 nm and particularly preferably 10 nm to 800 nm. Although not particularly limited, the shape of the thin film 310 may be a rectangular shape or a shape compatible with the shape of the discolored region or with the shape of the periphery thereof. In addition, an outer circumferential portion and/or an inside of the thin film 310 may be notched so that the thin film 310 is compatible with the shape of the discolored region or the periphery thereof.

The thin film 310 may be a sheet formed by a spin coating method, a roll-to-roll method, an LB method (Langmuir-Blodgett method), or the like or may also be a fiber sheet in which fibers formed by an electrospinning method or the like are folded and laminated to each other. As an example of the thin film 310, for example, there may be mentioned a polyester, such as a polyglycolic acid, a polylactic acid, a polycaprolactone, a poly(ethylene succinate), a poly(ethylene terephthalate), or a copolymer thereof; a polyether, such as a polyethylene glycol or a polypropylene glycol; a polyamide, such as a nylon, a polyglutamic acid, a polyaspartic acid, or a salt thereof; a polysaccharide, such as a pullulan, a cellulose, a starch, a chitin, a chitosan, an alginic acid, a hyaluronic acid, a cornstarch, or a salt thereof; a silicone, such as an acrylic silicone or a trimethylsiloxysilicate; an acrylic acid, such as an alkyl acrylate, a silicon acrylate, an acrylamide, or a copolymer thereof; a poly(vinyl alcohol); a polyurethane; a polycarbonate: a poly(acid anhydride); a polyethylene; a polypropylene; a porous coating sheet or a nanofiber sheet; or a silk. In view of biocompatibility, availability, and handleability, as the material of the thin film 310, for example, a polylactic acid, a cellulose (such as a carboxymethyl cellulose or a hydroxyethyl cellulose), a starch, a chitin, a chitosan, an alginic acid, a cornstarch, or a polyurethane is preferable.

For example, the first layer 320, the second layer 330, and the third layer 340 are each a layer primarily containing a colorant and a binder. The chromaticity and the lightness of each of the first layer 320, the second layer 330, and the third layer 340 may be adjusted by combination of colorants, the thickness, and the like. In addition, the first layer 320, the second layer 330, and the third layer 340 may further contain a film forming agent, various types of additives, and the like.

As an example of the colorant, for example, there may be mentioned an inorganic red pigment, such as iron titanate including iron oxide or iron hydroxide; an inorganic brown-based pigment such as γ-iron oxide; an inorganic yellow-based pigment, such as yellow iron oxide or yellow soil; an inorganic black pigment, such as black iron oxide or carbon black; an inorganic purple pigment, such as manganese violet or cobalt violet; an inorganic green pigment, such as chromium hydroxide, chromium oxide, cobalt oxide, or cobalt titanate; an inorganic blue-based pigment, such as Prussian blue (ferric ferrocyanide), navy blue (ultramarine blue), lapis lazuli, rock ultramarine, aluminum-cobalt oxide, aluminum-zinc-cobalt oxide, silicon-cobalt oxide, silicon-zinc-cobalt oxide, a cobalt pigment, smalt, cobalt blue, cobalt stannate, cobalt chromium blue, cobalt-aluminum-silicon oxide, or manganese blue; an organic blue pigment or blue dye, such as indigo, phthalocyanine, indanthrene blue, or a sulfonated compound thereof; various types of laked tar-based dyes, various types of laked natural dyes, or a powdered synthetic resin formed by compositing at least one of those powders mentioned above.

The first layer 320, the second layer 330, and the third layer 340 may contain either only one type of colorant or at least two types of colorants. In addition, the shapes of those colorants are not particularly limited, and for example, any one of a needle shape, an indeterminate shape, a spherical shape, and a plate shape may be used.

As an example of the binder, for example, there may be mentioned particles formed from a (meth)acrylic resin, such as an alkyl (meth)acrylate polymer, a styrene-(meth)acrylate copolymer, an alkyl (meth)acrylate-vinyl acetate copolymer, a (meth)acrylic acid-alkyl (meth)acrylate copolymer, or a (meth)acrylic acid-alkyl dimethicone polymer; a vinyl acetate polymer; or a vinylpyrrolidone-styrene copolymer. In addition, in the present disclosure, the (meth)acryl indicates an acryl, a methacryl, or a mixture of an acryl and a methacryl. Among those mentioned above, the binder is preferably particles (hereinafter, also simply referred to as "acrylic particles" in some cases) formed from a (meth)acrylic resin. When the binder is formed from acrylic particles, a fixing property of the colorant can be improved. The binder is further preferably formed from a (meth)acrylic resin having no skin irritation. Accordingly, the acrylic particles described above are preferably selected, for example, from components listed in the component display name list of cosmetics based on the Pharmaceutical Affairs Law of Japan, components in accordance the EU Cosmetics directive 76/768/EEC, and components listed International Cosmetic Ingredient Dictionary and Handbook (9th edition, January 1, 2002) by the US CTFA (Cosmetic, Toiletry and Fragrance Association, U.S.) and are preferably formed from an acrylic resin which has been used for known cosmetics.

As a particular example of the (meth)acrylic resin forming the acrylic particles, for example, there may be mentioned a homopolymer of a (meth)acrylic monomer, a copolymer of at least two types of (meth)acrylic monomers, or a copolymer of a (meth)acrylic monomer and another monomer. As an example of the above (meth)acrylic monomer, there may be mentioned acrylic acid, methyl acrylate, ethyl acrylate, acrylamide, n-propyl acrylate, n-butyl acrylate, isobutyl acrylate, octyl acrylate, 2-ethylhexyl acrylate, N,N-dimethylamino ethyl acrylate, acrylonitrile, methacrylic acid, ethyl methacrylate, methacrylamide, n-propyl methacrylate, n-butyl methacrylate, isobutyl methacrylate, 2-ethylhexyl methacrylate, octyl methacrylate, hydroxyethyl methacrylate, or N,N-dimethylamino ethyl methacrylate. In addition, as an example of the another monomer copolymerizable with the (meth)acrylic monomer, for example, styrene, vinyl acetate, a silicone macromer, a fluorine-based monomer, or an alkoxysilane unsaturated monomer may be mentioned.

When a content of the colorant is assumed as 10 parts by mass, a content of the binder contained in the first layer 320, the second layer 330, or the third layer 340 is preferably 0.5 to 10 parts by mass and more preferably 1.5 to 5.7 parts by mass. When the content of the binder with respect to the content of the colorant is in the range described above, the fixing property of the colorant is increased. In addition, when the content of the binder is in the range described above, the content of the colorant is likely to be relatively sufficient, and the first layer 320, the second layer 330, or the third layer 340 is able to have a desired color.

A method of applying an ink on the thin film by the image formation section 160 is not particularly limited and may be selected from known methods. As an example of the application method, for example, an ink jet method, a screen printing, an off-set printing, or a gravure printing may be mentioned. In consideration that on-demand printing and lamination printing are easily carried out, among those methods mentioned above, an ink jet method is preferable. Hereinafter, although the case in which a cosmetic ink is applied by an ink jet method will be described, the present disclosure is not limited to this method.

When printing is performed by an ink jet method, the structure of an ink jet device is not particularly limited and may be selected from known methods (such as a piezoelectric method, a thermal method, and an electrostatic method). Since heating is not required, among those methods mentioned above, an ink jet device using a piezoelectric element method is preferable.

In addition, when the layers are each formed, the image formation section 160 may applying the ink only one time or at least two times. When the ink is applied at least two times, drying may be performed every time after the ink is applied or may be collectively performed after the ink is applied at least two times.

A method to dry the ink is not particularly limited as long as a solvent (such as a higher alcohol or purified water which will be described later) of the ink can be removed. For example, the ink may be dried at room temperature and an atmospheric pressure or may be dried by heating to a predetermined temperature and/or by reducing the pressure. When the heating is performed, the temperature is preferably increased, for example, to 25°C to 50°C. When the heating is performed in the range described above, without degrading the thin film and a solid component in the ink, the drying can be efficiently performed. On the other hand, when the pressure is reduced, the pressure is preferably reduced to -0.1 to 0 MPa. When the pressure is reduced to this range, the ink can be efficiently dried.

Heretofore, although the image processing apparatus (makeup support system) 100 including the image formation section 160 has been described, the image processing apparatus according to the present disclosure may include no image formation section 160. For example, in the image processing apparatus according to the present disclosure, the image data produced by the image data production section 140 is transmitted to an exterior coating device or printing device, and this exterior coating device or printing device may form on a skin or a thin film, an image to correct the color of the discolored portion to the color of the normal portion.

Fig. 2 is a schematic view showing one example of the image processing apparatus (makeup support system) 100 including the image formation section 160 which forms the sheet 300. As shown in Fig. 2, the image processing apparatus 100 has a first device 170 which includes the image acquisition section 110, the image storage section 120, the image analysis section 130, the image data production section 140, and the information storage section 150 and a second device 180 which includes the image formation section 160. The first device 170 functions as a device to produce the image data, and the second device 180 functions as a device to form the sheet 300 based on the image data produced by the first device 170. The first device 170 and the second device 180 are connected to each other by wire communication or wireless communication.

While visible light is radiated from a lighting portion 111, the first device 170 photographs a face of a user 400 positioned at a front side of a display portion 113 by a camera 112 disposed in the vicinity of the display portion 113. In addition, in the first device 170, a face image 410 is produced by reversing a photographed image from left to right and is then displayed on the display portion 113. Accordingly, the user 400 is able to photograph the face image 410 while feeling as if looking in a mirror. In this example, the lighting portion 111, the camera 112, and the display portion 113 cooperatively function as the image acquisition section 110.

In addition, the first device 170 identifies at least one discolored region 420 from the face image 410 (or the face image before the reversal). In addition, the first device 170 produces the image data to produce the sheet 300 which makes a discolored portion of a skin 430 corresponding to a discolored region 420 of the identified face image 410 inconspicuous. In addition, the first device 170 transmits this image data to the second device 180 by wire communication or wireless communication.

Based on the image data transmitted from the first device 170, the second device 180 forms using various types of inks filled in ink tanks in combination, the sheet 300 by forming (lamination printing) the first layer 320, the second layer 330, and the third layer 340 on the thin film 310 to have a desired pattern.

The user 400 is able to make the discolored portion inconspicuous by adhering the sheet 300 thus formed on the discolored portion of the skin 430. In addition, when the first device 170 judges that no discolored regions 420 are present, the second device 180 may form a sheet including neither first layer 320 nor second layer 330, that is, a sheet 300 in which the third layer 340 is laminated on the thin film 310 to have an individual skeleton size. In this case, the absence of the discolored portions is displayed on the display portion 113, and the user 400 may be allowed to select whether no printing is performed or a printing of the third layer 340 corresponding to foundation is performed.

The first device 170 and the second device 180 are installed in, for example, a work, a cosmetics store, a beauty salon, a medical institution, a makeup room to adjust his or her appearance, an event site, or a private house. In addition, the first device 170 and the second device 180 each may also be a portable device which can be easily carried.

In addition, in Fig. 2, although the image processing apparatus (makeup support system) 100 in which the first device 170 and the second device 180 are separately provided has been described, the image processing apparatus according to the present disclosure is not limited thereto. For example, the image processing apparatus according to the present disclosure may include the image acquisition section 110, the image storage section 120, the image analysis section 130, the image data production section 140, the information storage section 150, and the image formation section 160 in one housing.

Heretofore, although the image processing apparatus (makeup support system) 100 according to the present disclosure has been described in detail with reference to the drawings, the function of the image processing apparatus 100 described above can be realized by a computer program.

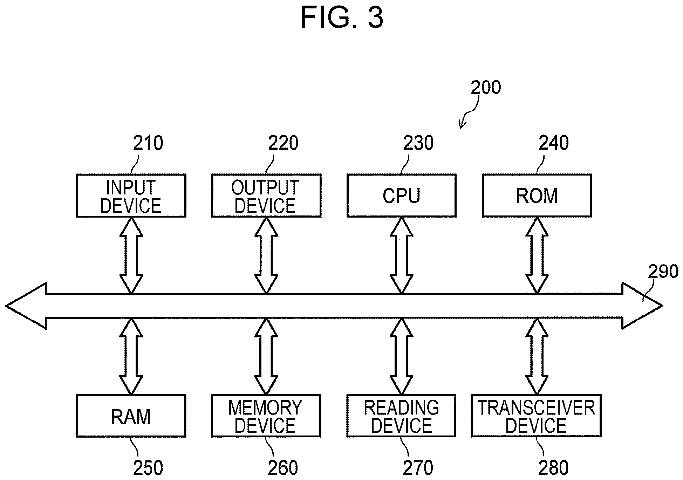

Fig. 3 is a view showing a hardware structure of a computer which realizes by a program, the functions of the image acquisition section 110, the image storage section 120, the image analysis section 130, the image data production section 140, and the information storage section 150 of the image processing apparatus 100. This computer 200 includes an input device 210, such as a keyboard, a mouse, a camera, and/or a touch pad; an output device 220, such as a display and/or a speaker; a CPU (central processing unit) 230, a ROM (read only memory) 240, a RAM (random access memory) 250, a memory device 260, such as a hard disc device and/or a SSD (solid state drive), a reading device 270 to read information from a recording medium, such as a DVD-ROM (digital versatile disk read only memory) and/or an USB (universal serial bus), and a transceiver device 280 to perform communication through a network, and the components described above are connected to each other with a bus 290.

In addition, from a recording medium in which a program to realize the function of the image processing apparatus 100 is recorded, the reading device 270 reads this program and stores it in the memory device 260. In addition, the transceiver device 280 communicates with a server device connected to the network and stores in the memory device 260, a program downloaded from the server device to realize the functions of the above devices.

In addition, the CPU 230 copies the program stored in the memory device 260 into the RAM 250, and commands contained in this program are sequentially read from the RAM 250 and carried out, so that the function of the image processing apparatus 100 can be realized.

### (INK COMPOSITION)

The first ink to form the first layer, the second ink to form the second layer, and the third ink to form the third layer (hereinafter, collectively referred to as "cosmetic ink" in some cases) which are used by the image formation section 160 are each an ink containing a colorant which adjusts the lightness (such as the L* value) and the chromaticity (such as the a* value and the b* value) of each layer. For example, the cosmetic ink contains a colorant, a binder, a higher alcohol, and purified water. The cosmetic ink may also contain, if needed, a film forming agent, various types of additives, and the like.

An example of the colorant contained in the cosmetic ink, for example, there may be mentioned an inorganic red pigment, such as iron titanate including iron oxide or iron hydroxide; an inorganic brown-based pigment such as γ-iron oxide; an inorganic yellow-based pigment, such as yellow iron oxide or yellow soil; an inorganic black pigment, such as black iron oxide or carbon black; an inorganic purple pigment, such as manganese violet or cobalt violet; an inorganic green pigment, such as chromium hydroxide, chromium oxide, cobalt oxide, or cobalt titanate; an inorganic blue-based pigment, such as Prussian blue (ferric ferrocyanide), navy blue (ultramarine blue), lapis lazuli, rock ultramarine, aluminum-cobalt oxide, aluminum-zinc-cobalt oxide, silicon-cobalt oxide, silicon-zinc-cobalt oxide, a cobalt pigment, smalt, cobalt blue, cobalt stannate, cobalt chromium blue, cobalt-aluminum-silicon oxide, or manganese blue; an organic blue pigment or blue dye, such as indigo, phthalocyanine, indanthrene blue, or a sulfonated compound thereof; various types of laked tar-based dyes, various types of laked natural dyes, or a powdered synthetic resin formed by compositing at least one of those powders mentioned above. Furthermore, the cosmetic ink may also contain a brightening agent, and as the brightening agent, for example, there may be mentioned a white pigment, such as titanium oxide, zinc oxide, cerium oxide, or barium sulfate; a white extender powder, such as talc, muscovite, phlogopite, lepidolite, biotite, synthetic mica, silk mica (sericite), synthetic sericite, kaolin, silicon carbide, bentonite, smectite, silicic anhydride, aluminum oxide, magnesium oxide, zirconium oxide, antimony oxide, diatomite, aluminum silicate, magnesium aluminometasilicate, calcium silicate, barium silicate, magnesium silicate, calcium carbonate, magnesium carbonate, hydroxyapatite, or boron nitride; a glitter powder, such as calcium aluminum borosilicate, titanium dioxide coated mica, powdered titanium dioxide coated glass, titanium dioxide coated bismuth oxychloride, titanium dioxide coated mica, titanium dioxide coated talc, iron oxide coated mica, iron oxide coated micaceous titanium, powdered iron oxide coated glass, navy blue treated micaceous titanium, carmine treated micaceous titanium, bismuth oxychloride, fish scale flake, powdered poly(ethylene terephthalate)-aluminum-epoxy laminate, or powdered poly(ethylene terephthalate)-polyolefin laminate film; an organic low molecular weight powder such as N-acyllysine; a natural organic powder, such as a silk powder or a cellulose powder; a metal powder, such as an aluminum powder, a gold powder, or a silver powder; a composite powder, such as fine titanium oxide coated micaceous titanium, fine zinc oxide coated micaceous titanium, barium sulfate coated micaceous titanium, titanium oxide-containing silicon dioxide, or zinc oxide-containing silicon dioxide.

As an example of the binder contained in the cosmetic ink, for example, there may be mentioned particles formed from a (meth)acrylic resin, such as an alkyl (meth)acrylate polymer, a styrene-(meth)acrylate copolymer, an alkyl (meth)acrylate-vinyl acetate copolymer, a (meth)acrylic acid-alkyl (meth)acrylate copolymer, or a (meth)acrylic acid-alkyl dimethicone polymer; a vinyl acetate polymer; or a vinylpyrrolidone-styrene copolymer. In addition, in the present disclosure, the (meth)acryl indicates an acryl, a methacryl, or a mixture of an acryl and a methacryl. Among those mentioned above, the binder is preferably particles (hereinafter, also simply referred to as "acrylic particles" in some cases) formed from a (meth)acrylic resin. When the binder is formed from acrylic particles, the fixing property of the colorant can be improved. The binder is further preferably formed from a (meth)acrylic resin having no skin irritation. Accordingly, the acrylic particles described above are preferably selected, for example, from components listed in the component display name list of cosmetics based on the Pharmaceutical Affairs Law of Japan, components in accordance the EU Cosmetics directive 76/768/EEC, and components listed International Cosmetic Ingredient Dictionary and Handbook (9th edition, January 1, 2002) by the US CTFA (Cosmetic, Toiletry and Fragrance Association, U.S.) and are preferably formed from an acrylic resin which has been used for known cosmetics.

As a particular example of the (meth)acrylic resin forming the acrylic particles, for example, there may be mentioned a homopolymer of a (meth)acrylic monomer, a copolymer of at least two types of (meth)acrylic monomers, or a copolymer of a (meth)acrylic monomer and another monomer. As an example of the above (meth)acrylic monomer, for example, there may be mentioned acrylic acid, methyl acrylate, ethyl acrylate, acrylamide, n-propyl acrylate, n-butyl acrylate, isobutyl acrylate, octyl acrylate, 2-ethylhexyl acrylate, N,N-dimethylamino ethyl acrylate, acrylonitrile, methacrylic acid, ethyl methacrylate, methacrylamide, n-propyl methacrylate, n-butyl methacrylate, isobutyl methacrylate, 2-ethylhexyl methacrylate, octyl methacrylate, hydroxyethyl methacrylate, or N,N-dimethylamino ethyl methacrylate. In addition, as an example of the another monomer copolymerizable with the (meth)acrylic monomer, for example, styrene, vinyl acetate, a silicone macromer, a fluorine-based monomer, or an alkoxysilane unsaturated monomer may be mentioned.

The type of higher alcohol is not particularly limited as long as having at least three carbon atoms and being compatible with purified water. The higher alcohol functions as a solvent of the cosmetic ink. In addition, after the cosmetic ink is applied, the higher alcohol is absorbed in the thin film or is vaporized. The number of carbon atoms of the higher alcohol is preferably 3 to 5 and more preferably 3 or 4. When the number of carbon atoms is in the range described above, the higher alcohol is likely to be compatible with purified water. In addition, in order to stably apply the cosmetic ink, the higher alcohol preferably includes a trivalent alcohol. As long as having no skin irritation, although the type of trivalent alcohol is not particularly limited, in view of biological safety and ink stability, glycerin is preferable.

In addition, the higher alcohol may also include a divalent alcohol or a monovalent alcohol. As an example of the divalent alcohol, for example, diethylene glycol, propylene glycol, 1,3-propanediol, butylene glycol, or hexanediol may be mentioned. As an example of the monovalent alcohol, for example, propanol, isopropanol, or butyl alcohol may be mentioned. In order to improve the wettability of the cosmetic ink to the thin film, the higher alcohol preferably contains a divalent alcohol and particularly preferably contains propylene glycol.

The purified water also functions as a solvent of the cosmetic ink and is absorbed in the thin film or vaporized after the cosmetic ink is printed. The type of purified water is not particularly limited as long as being generally used for cosmetics. The purified water may be water purified by various types of methods, such as a distillation and ion exchange or may be, for example, hot spring water, deep water, or steam distilled water of plants.

The film forming agent is a compound which enhances a film forming property (such as a drying property) of the cosmetic ink. In this specification, the "film forming agent" is assumed as a compound dispersible in water at room temperature (however, a component corresponding to the binder described above is excluded). In the cosmetic ink, either only one type of film forming agent or at leas two types thereof may be contained.

As the film forming agent, a compound which can be dispersed or dissolved in a higher alcohol and/or in purified water may be used, and for example, at least one compound selected from the group consisting of an acrylic polymer, a polysaccharide-based polymer, a sugar alcohol, a sterol, an ester, and a modified cornstarch may be used. The cosmetic ink may contain either only one type of film forming agent or at least two types thereof. When the film forming agent is a compound selected from the above group, a coating film formed from the cosmetic ink is very rapidly dried.

In addition, an HLB value of the film forming agent is preferably 8 o more and more preferably 8 to 19. When the HLB value of the film forming agent is 8 or more, the film forming agent is likely to be uniformly dispersed or dissolved in a higher alcohol or purified water. The HLB value is an index showing a ratio between relative affinities with respect to two types of liquids in an oil-water system, and a higher HLB value indicates a higher affinity to water. In addition, the HLB value of the present disclosure is assume to be calculated by Griffin method.

The film forming agent is also preferably a material having no skin irritation. As an example of the acrylic polymer described above, for example, there may be mentioned an acrylic acid alkyl copolymer, a 2-amino-2-methyl-1-propanol salt (hereinafter, also referred to as "AMP") of an acrylic acid alkyl copolymer, a sodium salt (hereinafter, also referred to as "Na") of an acrylic acid alkyl copolymer, an acrylic acid alkyl copolymer-ammonium, an acrylic acid-acrylic acid alkyl copolymer, an acrylic acid alkyl-diacetone acrylamide copolymer, an acrylic acid alkyl-diacetone acrylamide copolymer AMP, a 2-amino-2-methyl-1,3-propanediol salt (hereinafter, also referred to as "AMPD") of an acrylic acid alkyl-diacetone acrylamide copolymer, an acrylic acid hydroxyethyl-acrylic acid methoxyethyl copolymer, an acrylic acid hydroxyethyl-acrylic acid butyl-acrylic acid methoxyethyl copolymer, an acrylate-acryl acid alkyl (carbon atoms: 1 to 18)-alkyl (carbon atoms: 1 to 8) acrylamide copolymer AMP, an acryl acid alkyl-octyl acrylamide copolymer, an acrylate-t-butyl acrylamide copolymer, an acrylate acrylic acid ethylhexyl copolymer, an acrylate copolymer, an acrylate copolymer AMP, an acrylate copolymer Na, a polyurethane-14-acrylate copolymer AMP, a vinyl acetate-maleic acid butyl-acrylic acid isobororyl copolymer, a styrene-acrylic acid alkyl copolymer, a styrene-acrylate copolymer, a styrene-acrylic acid amide copolymer, polyurethane-1 (INCI name: compound represented by POLYURETHANE-1), polyacrylate-22 (INCI name: compound represented by : POLYACRYLATE-22), a tricontanyl polyvinyl pyrrolidone (PVP), an (eicosene/vinyl pyrrolidone) copolymer, or a (vinyl pyrrolidone/hexadecene) copolymer.

In addition, as an example of the polysaccharide-based polymer, for example, there may be mentioned gum arabic, glucan, succinoglycan, carrageenan, karaya gum, tragacanth gum, guar gum, locust bean gum, galactomannan gum, xanthan gum, starch, carob gum, quince seed (marmelo), casein, dextrin, gelatin, sodium pectinate, sodium alginate, methyl cellulose, ethyl cellulose, carboxymethyl cellulose, hydroxyethyl cellulose, hydroxypropyl cellulose, crystalline cellulose, chloride O- [2-hydroxy-3- (trimethylammonio)propyl] hydroxyethyl cellulose, chloride O- [2-hydroxy-3- (trimethylammonio)propyl] guar gum, chloride O- [2-hydroxy-3- (trimethylammonio)propyl] locust bean gum, chloride hydroxypropyltrimonium starch, glyceryl glucoside, glycosyl trehalose, tremella fuciformis, or dextrin isostearate.

As an example of the sugar alcohol, for example, there may be mentioned sorbitol, maltitol, or glucose. As the sterol, any compound having a sterol skeleton may be used, and as an example thereof, for example, there may be mentioned a phytosterol, such as campesterol, campestanol, brassicasterol, 22-dehydrocampesterol, stigmasterol, stigmastanol, 22-dihydrospinasterol, 22-dehydrostigmastanol, 7-dehydrostigmasterol, sitosterol, tirucallol, euphol, fucosterol, isofucosterol, codisterol, clionasterol, poriferasterol, clerolosterol, 22-dehydroclerosterol, fungisterol, condrillasterol, avenasterol, vernosterol, or polynastanol; a zoosterol, such as cholesterol, dihydrocholesterol, cholestanol, coprostanol, epicoprosterol, epicoprostanol, 22-dehydrocholesterol, desmosterol, 24-methylenecholesterol, lanosterol, 24,25-dihydrolanosterol, norlanosterol, spinasterol, dihydroagnosterol, agnosterol, lophenol, or lathosterol; a fungal sterol, such as dehydroergosterol, 22,23-dihydroergosterol, episterol, ascosterol, or fecosterol; or a hydrogenated product thereof.

As an example of the ester, for example, there may be mentioned a dipentaerythritol fatty acid ester, such as dipentaerythrityl hexa(hydroxystearic acid/stearic acid/rosin acid), dipentaerythrityl (hydroxystearic acid/stearic acid /rosin acid), dipentaerythrityl hexahydroxystearate, dipentaerythrityl tetra(hydroxystearic acid/isostearic acid), or dipentaerythrityl (hydroxystearic acid/isostearic acid); a hydrogenated castor oil fatty acid ester, such as stearic acid hydrogenated castor oil, isostearic acid hydrogenated castor oil, or hydroxystearic acid hydrogenated castor oil; a cholesterol fatty acid ester such as cholesteryl hydroxystearate; a phytosterol fatty acid ester, such as phytosteryl oleate or phytosteryl macadamia nut oil fatty acid ester; a hydrogenated vegetable oil, such as hydrogenated coconut oil or hydrogenated palm oil; dimer dilinoleic acid (phytosteryl/isosteryl/cetyl/stearyl/behenyl); sucrose pentahydroxystearate; lauroyl glutamic acid di(octyldodecyl/phytosteryl/behenyl).

As the modified cornstarch, as long as the aim and the effect of the present disclosure are not deteriorated, a compound obtained such that a cornstarch is modified by an arbitrary compound may be used, and for example, a hydroxypropyl modified starch obtained by a reaction between a cornstarch and 3-(dodecenyl)dihydro-2,5-furandione may also be used.

Among those mentioned above, since the drying property of the cosmetic ink can be improved, for example, an acrylate copolymer, an acrylate (ethylhexyl acrylate) copolymer, a polyurethane-14/acrylate copolymer AMP, an acrylic aid alkyl copolymer-ammonium, a dimer dilinoleic acid dimer dilinoleyl bis(behenyl/isostearyl/phytosteryl)-triglyceryl hydrogenated rosin acid, a xanthan gum cross polymer-hydroxyethyl cellulose, a tremella fuciformis polysaccharide, a modified corn starch, or dextrin isostearate is preferable, and an acrylate copolymer, an acrylate (ethylhexyl acrylate) copolymer, a polyurethane-14/acrylate copolymer AMP, an acrylic acid alkyl copolymer-ammonium, a dimer dilinoleic acid dimer dilinoleyl bis(behenyl/isostearyl/phytosteryl)-triglyceryl hydrogenated rosin acid, a xanthan gum cross polymer-hydroxyethyl cellulose, or a tremella fuciformis polysaccharide is more preferable.

In addition, a content of the film forming agent contained in 100 parts by mass of the cosmetic ink is preferably 20 parts by mass or less and more preferably 0.3 to 5 parts by mass. When the content of the film forming agent is 0.3 parts by mass or more, as described above, the drying property of the cosmetic ink is improved. On the other hand, when the content of the film forming agent is excessively large, although the viscosity of the cosmetic ink is excessively increased in some cases, when the content is 20 parts by mass or less, the cosmetic ink can be made to have a viscosity at which printing is easily performed by various application methods or printing methods.

In addition, when the cosmetic ink is prepared, in general, after being dissolved in a solvent to form a solution, the film forming agent is mixed with a colorant, a reflective material, a higher alcohol, purified water, and/or the like. As the solvent to be used in this case is also preferably a solvent having no skin irritation, and the higher alcohol or purified water described above is preferable.

In addition, as long as the aim and the effect of the present disclosure are not deteriorate, for example, a water-soluble polymer (polymer not corresponding to the film forming agent described above) may also be contained in the cosmetic ink to bond a reflective material, a colorant, and/or the like to an object to be printed.

The various types of additives are also preferably compounds having no skin irritation. As an example of the various types of additives, for example, there may be mentioned a surfactant, a pH adjuster, a thickening agent, an ultraviolet absorber, an ultraviolet scatter, an antiseptic/antifungal agent, a deoxidizer, an oxidation inhibitor, a corrosion inhibitor, an antifading agent, a defoaming agent, a fragrance, or a solvent other than a higher alcohol and purified water.

### (OPERATION OF IMAGE PROCESSING APPARATUS)

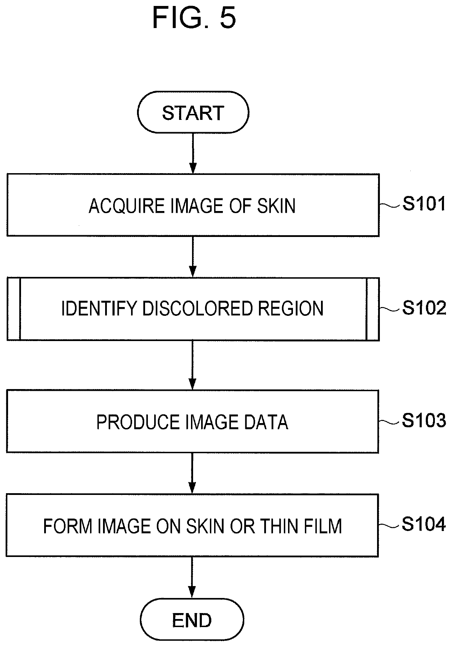

Next, with reference to a flowchart of Fig. 5, an operation of the image processing apparatus 100 will be described.

First, the image acquisition section 110 acquires an image of a skin including a discolored portion, stores the image in the image storage section 120, and further transmits the image to the image analysis section 130 (S101).

Next, the image analysis section 130 identifies a discolored region corresponding to the discolored portion from the image acquired by the image acquisition section 110 (S102). In more particular, the image analysis section 130 identifies a color and a shape of the discolored region corresponding to the discolored portion from the image acquired by the image acquisition section 110 and also identifies a color of a normal region (non-discolored region) present along a periphery of the discolored region. The identified information is transmitted to the image data production section 140.

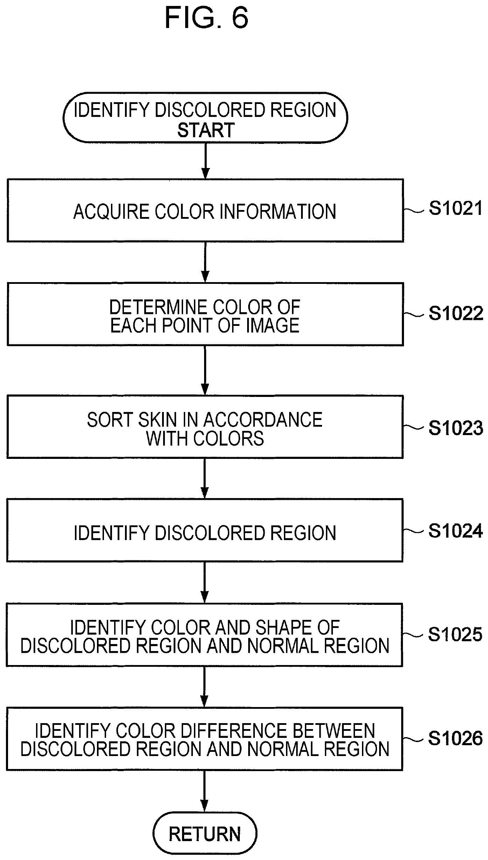

Fig. 6 is a flowchart showing one example of processing of the image analysis section 130 in Step S102.

First, using a color chart obtained before or at the same time the image acquisition section 110 acquires the image of the skin, the image analysis section 130 acquires color information to correct the color of the image of the skin (S1021).

Next, based on the color information obtained in Step S1021, the image analysis section 130 performs color correction of the image of the skin acquired by the image acquisition section 110 and determines the color of each point of the skin (S1022).

Subsequently, the image analysis section 130 sorts the image of the skin in accordance with colors (S1023). In more particular, the image analysis section 130 sets a plurality of ranges of the chromaticity (such as the a* value and the b* value) and/or the lightness (such as the L* value), and the image of the skin is sorted in accordance with the respective ranges. For example, when the image analysis section 130 performs the sorting in accordance with the lightness (such as the L* value), the image analysis section 130 sorts the image of the skin into a region (corresponding to the normal region (non-discolored region) corresponding to the normal portion) in which the L* value is less than the first threshold value and higher than or equal to a third threshold value, a region (corresponding to the discolored region having a high lightness corresponding to a discolored portion (such as a vitiligo) having a high lightness) in which the L* value is the first threshold value or more, and a region (corresponding to a discolored region having a low lightness corresponding to a discolored portion (such as a spot) having a low lightness) in which the L* value is less than the third threshold value.

Next, the image analysis section 130 identifies from the image, a discolored region having a high lightness corresponding to a discolored portion having a high lightness (S1024). As described above, the method of the image analysis section 130 to identify the discolored region is not particularly limited. For example, the image analysis section 130 may identify the discolored region by allowing the user 400 to designate a range of the discolored region 420 in the image 410 using the display portion 113, such as the touch panel-equipped liquid crystal display described above, also functioning as the input portion (see Fig. 2). Alternatively, the image analysis section 130 may identify in the image, a region in which the lightness (such as the L* value) is the predetermined first threshold value or more as the discolored region. In this case, when the region in which the lightness (such as the L* value) is the first threshold value or more is present, although the image analysis section 130 may immediately identify this region as the discolored region, in order to improve the identification accuracy of the discolored region, only when the other conditions are also satisfied, the region described above may be identified as the discolored region.

Subsequently, the image analysis section 130 identifies the color (the lightness and the chromaticity) and the shape of the identified discolored region and the color (the lightness and the chromaticity) of the peripheral normal region (S1025).

Finally, the image analysis section 130 identifies the color difference between the discolored region and the normal region (S1026).

Subsequently, again with reference to the flowchart of Fig. 5, the image data production section 140 produces image data to correct the color of the discolored portion to the color of the normal portion based on the information of the color of the normal region of the image corresponding to the normal portion and the color of the discolored region acquired by the image acquisition section 110 (S103). The produced image data is transmitted to the image formation section 160. In this case, the image data production section 140 sets an L* value of a region of the first layer corresponding to the discolored region to a value lower than the L* value of the normal region by higher than or equal to 5 and lower than or equal to 20 and preferably by higher than or equal to 10 and lower than or equal to 20. In many cases, in the region of the first layer corresponding to the discolored region, although the L* value may be uniformly set to a value lower than the L* value of the normal region by higher than or equal to 10 and lower than or equal to 20, for example, when the lightness L* of the normal region is 50 or less, the L* value of the region of the first layer corresponding to the discolored region may be set to a value lower than the L* value of the normal region by higher than or equal to 5 and lower than or equal to 10.

Finally, based on the image data produced by the image formation section 160, the image formation section 160 forms the image on a skin or a thin film (s104). In particular, the image formation section 160 sequentially applies on the skin or the thin film, a first ink to form the first layer, a second ink to form the second layer, and a third ink to form the third layer and then dries those inks, so that the first layer located at a skin side, the second layer disposed on the first layer, and the third layer disposed on the second layer are formed. The drying may be collectively performed after all the first ink, the second ink, and the third ink are applied or may be performed at each time when the first ink, the second ink, or the third ink is applied. In addition, the first ink, the second ink, and the third ink may be applied while the skin or the thin film is heated, so that the application of the ink and the drying thereof are simultaneously performed. When the drying is performed every time after the first ink, the second ink, or the third ink is applied or is performed in parallel with the application thereof, the ink adhered to the skin or the thin film is rapidly dried, and hence, color irregularity caused by mixing between adjacent inks can be prevented from being generated.

According to the procedure described above, the image processing apparatus 100 is able to produce the image data to correct the color of the discolored portion having a lightness higher than that of the normal portion of the skin to the color of the normal portion and, based on this image data, is also able to form the image to correct the color of the discolored portion to the color of the normal portion on the skin or the thin film.

### (EFFECTS)

As has thus been described, the image processing apparatus 100, the image processing method, and the sheet 300 according to the present disclosure are each able to make the discolored portion having a lightness higher than that of the periphery thereof inconspicuous without generating a thick coating feeling.

### EXAMPLES

Hereinafter, with reference to Examples, the present disclosure will be described. The scope of the present disclosure is not understood to be limited to Examples.

### [EXPERIMENT 1]

In Experiment 1, an effect in which an L* value in a region of a first layer corresponding to a discolored region was set to a value lower than an L* value of a normal region by higher than or equal to 5 and lower than or equal to 20 was confirmed.

### (1) FORMATION OF VITILIGO PATTERN

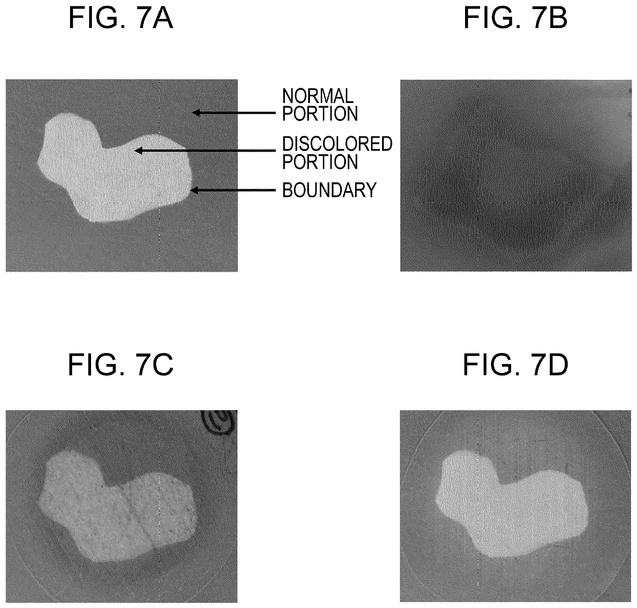

A white cosmetic material was sprayed on a synthetic leather to form a vitiligo pattern. Fig. 7A is a photo of the formed vitiligo pattern. As shown in Fig. 7A, a region colored white by the cosmetic material corresponds to a discolored portion. The discolored portion is surrounded by a normal portion having a pale orange color similar to a color of a skin of a yellow race person. As apparent from Fig. 7A, a lightness (L* value) of the discolored portion is higher than a lightness (L* value) of the normal portion.

### (2) FORMATION OF SHEET

Inks containing various types of colorants were applied by a spray method on a thin film formed form a polylactic acid having a thickness of 200 nm, so that three types of sheets, that is, a first sheet, a second sheet, and a third sheet, were formed. Those sheets each included only a first layer without including a second layer and a third layer. Those sheets were each set so that the L* value, the a* value, and the b* value of a region corresponding to the discolored portion were set as described below.

### • FIRST SHEET

L* value: lower than the L* value of the normal portion by 5 to 20.
a*' value: substantially the same as the a* value of the normal portion.
b*' value: substantially the same as the b* value of the normal portion.

### • SECOND SHEET

L* value: substantially the same as the L* value of the normal portion.
a*' value: higher than the a* value of the normal portion by 5 to 25.
b*' value: substantially the same as the b* value of the normal portion.

### • THIRD SHEET

L* value: substantially the same as the L* value of the normal portion.
a*' value: substantially the same as the a* value of the normal portion.
b*' value: lower than the b* value of the normal portion by 5 to 25.

### (3) EVALUATION OF SHEET

The first sheet, the second sheet, or the third sheet was placed on the discolored portion having the vitiligo pattern, and the change in appearance of the discolored portion was observed. Fig. 7B is a photo of the vitiligo pattern on which the first sheet was placed, Fig. 7C is a photo of the vitiligo pattern on which the second sheet was placed, and Fig. 7D is a photo of the vitiligo pattern on which the third sheet was placed.

As shown in Fig. 7B, in the vitiligo pattern on which the first sheet having a low lightness was placed, the discolored portion was hardly observed, and the boundary between the discolored portion the normal portion was also hardly observed. On the other hand, as shown in Fig. 7C and Fig. 7D, in the vitiligo pattern on which the second sheet or the third sheet, in each of which the chromaticity was changed, was placed, the discolored portion and the boundary between the discolored portion and the normal portion were clearly observed.

From the results described above, it was found that when the L* value of the region of the first layer corresponding to the discolored portion (discolored region) was set to a value lower than the L* value of the normal portion (normal region) by higher than or equal to 5 and lower than or equal to 20, the discolored portion having a lightness higher than that of the normal portion could be effectively shielded.

### [EXPERIMENT 2]

In Experiment 2, an effect in which a discolored portion having a lightness higher than that of a normal portion could be shielded by the sheet according to the present disclosure was confirmed.

### (1) FORMATION OF SHEET

Inks containing various types of colorants were applied by an ink jet method on a thin film formed form a polylactic acid having a thickness of 200 nm, so that two types of sheets, that is, a fourth sheet and a fifth sheet, were formed. Those sheets each included three layers, that is, a first layer, a second layer, and a third layer. In the sheets described above, the L* value, the a* value, and the b* value of a region of each layer corresponding to the discolored portion were set as described below. Although the lightness and the chromaticity of the first layer of the fifth sheet were each set in a different manner from that of the first layer of the fourth sheet, the lightness and the chromaticity of the second layer of the fifth sheet were each set in the same manner as the second layer of the fourth sheet, and the lightness and the chromaticity of the third layer of the fifth sheet were each also set in the same manner as the third layer of the fourth sheet.

### • FOURTH SHEET

L* value of the first layer: substantially the same as the L* value of the normal portion.
a*' value of the first layer: higher than the a* value of the normal portion by 0.5 to 15.
b*' value of the first layer: substantially the same as the b* value of the normal portion.
L* value of the second layer: intermediate value between the L* value of the first layer and the L* value of the third layer.
a*' value and b* value of the second layer: substantially the same as the a* value and the b* value of the normal portion.
L* value of the third layer: substantially the same as the L* value of the normal portion.
a*' value and b* value of the third layer: substantially the same as the a* value and the b* value of the normal portion.

### • FIFTH SHEET

L* value of the first layer: lower than the L* value of the normal portion by 5 to 20.
a*' value and b* value of the first layer: substantially the same as the a* value and the b* value of the normal portion.
L* value of the second layer: intermediate value between the L* value of the first layer and the L* value of the third layer.
a*' value and b* value of the second layer: substantially the same as the a* value and the b* value of the normal portion.
L* value of the third layer: substantially the same as the L* value of the normal portion.
a*' value and b* value of the third layer: substantially the same as the a* value and the b* value of the normal portion.

### (2) EVALUATION OF SHEET

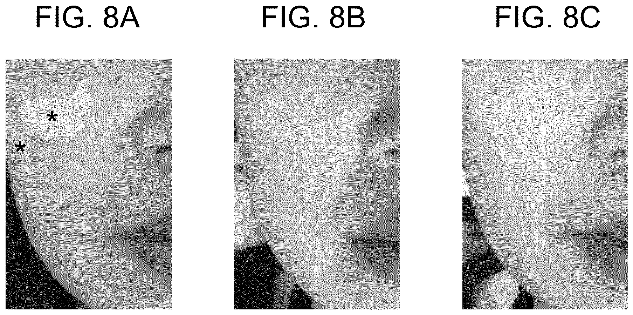

A white cosmetic material was sprayed on a skin in the vicinity of a cheekbone of a yellow race person, so that discolored portions were artificially formed. Fig. 8A is a photo of a face having the discolored portions. As shown by two asterisks in Fig. 8A, in this experiment, two discolored portions were formed. As apparent from Fig. 8A, the lightness (L* value) of the discolored portion is higher than the lightness (L* value) of the normal portion, and the boundary between the discolored portion and the normal portion can be clearly confirmed.

The fourth sheet or the fifth sheet was adhered to the skin having the discolored portions, and the change in appearance of the discolored portions was observed. Fig. 8B is a photo of the face to which the fourth sheet is adhered, and Fig. 8C is a photo of the face to which the fifth sheet is adhered.

As shown in Fig. 8B, even after the fourth sheet in which the chromaticity of the first layer was changed was adhered, the discolored portion and the boundary between the discolored portion and the normal portion were confirmed. On the other hand, as shown in Fig. 8C, when the fifth sheet in which the lightness of the first layer was set lower was adhered, the discolored portion and the boundary between the discolored portion and the normal portion were hardly confirmed.

From the results described above, it was found that the sheet according to the present disclosure was able to effectively shield the discolored portion having a lightness higher than that of the normal portion.

### INDUSTRIAL APPLICABILITY

The image processing apparatus, the image processing method, and the sheet of the present disclosure are able to make a discolored portion having a lightness higher than that of a periphery thereof inconspicuous without generating a thick coating feeling. Hence, the image processing apparatus, the image processing method, and the sheet of the present disclosure are each effective, for example, as a cosmetic technique which corrects a vitiligo or the like.

### Reference Signs List

- 100: image processing apparatus (makeup support system)
- 110: image acquisition section
- 120: image storage section
- 130: image analysis section
- 140: image data production section
- 150: information storage section
- 160: image formation section
- 170: first device
- 180: second device
- 200: computer
- 210: input device
- 220: output device
- 230: CPU
- 240: ROM
- 250: RAM
- 260: memory device
- 270: reading device
- 280: transceiver device
- 290: bus
- 300: sheet
- 310: thin film
- 320: first layer
- 330: second layer
- 340: third layer
- 400: user
- 410: image
- 420: discolored region
- 430: skin

## Claims

1. An image processing apparatus which produces image data to correct a color of a discolored portion having a lightness higher than that of a normal portion of a skin to a color of the normal portion, the image processing apparatus comprising:
an image analysis section which identifies a discolored region corresponding to the discolored portion from a photographed image of the discolored portion; and
an image data production section which produces, based on information of a color of a normal region of the image corresponding to the normal portion and a color of the discolored region, the image data to correct the color of the discolored portion to the color of the normal portion,
wherein the image data includes a first layer located at a skin side, a second layer located on the first layer, and a third layer located on the second layer, and
the image data production section sets an L* value of a region of the first layer corresponding to the discolored region to a value lower than an L* value of the normal region by higher than or equal to 5 and lower than or equal to 20.

2. The image processing apparatus according to claim 1,
wherein the image data production section sets a chromaticity of a region of the second layer corresponding to the discolored region to a chromaticity substantially having a complementary color relationship with that of the region of the first layer corresponding to the discolored region.

3. The image processing apparatus according to claim 1 or 2,
wherein the image data production section sets a lightness of a region of the second layer corresponding to the discolored region to a lightness between a lightness of the region of the first layer corresponding to the discolored region and a lightness of a region of the third layer corresponding to the discolored region.

4. The image processing apparatus according to claim 1 or 2,
wherein the image data production section sets a lightness of a region of the second layer corresponding to the discolored region to a lightness higher than a lightness of the region of the first layer corresponding to the discolored region and a lightness of a region of the third layer corresponding to the discolored region.

5. The image processing apparatus according to any one of claims 1 to 4,
wherein the image data production section sets a lightness and a chromaticity of a region of the third layer corresponding to the discolored region to substantially the same as a lightness and a chromaticity of the normal region.

6. The image processing apparatus according to any one of claims 1 to 5,
wherein the image data production section sets in the first layer, a gradation region between the region corresponding to the discolored region and a region corresponding to the normal region in which a color is continuously changed from a color of the region corresponding to the discolored region to a color of the region corresponding to the normal region.

7. The image processing apparatus according to claim 6,
wherein the image data production section sets in the second layer or the third layer, a gradation region between a region corresponding to the discolored region and a region corresponding to the normal region in which a color is continuously changed from a color of the region corresponding to the discolored region to a color of the region corresponding to the normal region.

8. The image processing apparatus according to claim 7,
wherein a width of the gradation region in the second layer or the third layer is larger than a width of the gradation region in the first layer.

9. The image processing apparatus according to claim 7 or 8,
wherein an inner edge of the gradation region in the second layer or the third layer is located outside than an inner edge of the gradation region in the first layer.

10. The image processing apparatus according to any one of claims 1 to 9,
wherein the image analysis section identifies a region in which an L* value is a predetermined first threshold value or more as the discolored region.

11. The image processing apparatus according to claim 10,
wherein when at least 30% of an outer edge of the region in which the L* value is the first threshold value or more is surrounded by a region in which the L* value is less than the first threshold value, the image analysis section identifies the region in which the L* value is the first threshold value or more as the discolored region.

12. The image processing apparatus according to claim 10 or 11,
wherein when a plurality of regions in each of which the L* value is less than the first threshold value is not present at intervals of a predetermined second threshold value or less in the region in which the L* value is the first threshold value or more, the image analysis section identifies the region in which the L* value is the first threshold value or more as the discolored region.

13. The image processing apparatus according to any one of claims 1 to 12,
further comprising an image formation section which forms an image on a skin or a thin film based on the image data.

14. The image processing apparatus according to claim 13,
wherein the image formation section forms the image on the skin by a spray coating.

15. The image processing apparatus according to claim 13,
wherein the image formation section forms the image on the skin by an ink jet printing.

16. The image processing apparatus according to claim 13,
wherein the image formation section prints the image on a thin film to be adhered to a skin.

17. The image processing apparatus according to any one of claims 1 to 16,
further comprising an image acquisition section which acquires an image of a skin.

18. An image processing method which produces image data to correct a color of a discolored portion having a lightness higher than that of a normal portion of a skin to a color of the normal portion, the method comprising:
a step of identifying a discolored region corresponding to the discolored portion from a photographed image of the discolored portion; and
a step of producing, based on information of a color of a normal region of the image corresponding to the normal portion and a color of the discolored region, image data to correct the color of the discolored portion to the color of the normal portion,
wherein the image data includes a first layer located at a skin side, a second layer located on the first layer, and a third layer located on the second layer, and
in the step of producing image data, an L* value of a region of the first layer corresponding to the discolored region is set to a value lower than an L* value of the normal region by higher than or equal to 5 and lower than or equal to 20.

19. A sheet which corrects a color of a discolored portion having a lightness higher than that of a normal portion of a skin to a color of the normal portion, the sheet comprising:
a thin film having one surface to be adhered to the skin;
a first layer disposed on the other surface of the thin film;
a second layer disposed on the first layer; and
a third layer disposed on the second layer,
wherein an L* value of a region of the first layer corresponding to the discolored portion is lower than an L* value of the normal portion by higher than or equal to 5 and lower than or equal to 20, and
a lightness and a chromaticity of a region of the third layer corresponding to the discolored portion are substantially the same as a lightness and a chromaticity of the normal portion.

20. The sheet according to claim 19,
wherein a chromaticity of a region of the second layer corresponding to the discolored portion substantially has a complementary color relationship with that of the region of the first layer corresponding to the discolored portion.

21. The sheet according to claim 19 or 20,
wherein a lightness of a region of the second layer corresponding to the discolored portion is a lightness between a lightness of the region of the first layer corresponding to the discolored portion and the lightness of the region of the third layer corresponding to the discolored portion.

22. The sheet according to claim 19 or 20,
wherein a lightness of a region of the second layer corresponding to the discolored portion is a lightness higher than a lightness of the region of the first layer corresponding to the discolored portion and the lightness of the region of the third layer corresponding to the discolored portion.

23. The sheet according to any one of claims 18 to 22,
wherein the first layer includes a gradation region disposed between the region corresponding to the discolored portion and a region corresponding to the normal portion in which a color is continuously changed from a color of the region corresponding to the discolored portion to a color of the region corresponding to the normal portion.

24. The sheet according to claim 23,
wherein the second layer or the third layer includes a gradation region disposed between a region corresponding to the discolored portion and a region corresponding to the normal portion in which a color is continuously changed from a color of the region corresponding to the discolored portion to a color of the region corresponding to the normal portion.

25. The sheet according to claim 24,
wherein a width of the gradation region of the second layer or the third layer is larger than a width of the gradation region of the first layer.

26. The sheet according to claim 24 or 25,
wherein an inner edge of the gradation region of the second layer or the third layer is located outside than an inner edge of the gradation region of the first layer.
